# EUROPEAN PATENT SPECIFICATION

(11) **EP 2 892 463 B1**
(45) Date of publication and mention of the grant of the patent: **12.07.2017**
(21) Application number: 13725431.4
(22) Date of filing: 21.05.2013
(51) Int. Cl.: A61F 2/08

(54) **SYNTHETIC LIGAMENT ASSEMBLY**
SYNTHETISCHE LIGAMENTANORDNUNG
ENSEMBLE LIGAMENT SYNTHÉTIQUE

(30) Priority: 11.06.2012 GB 201210203
(43) Date of publication of application: 15.07.2015
(73) Proprietor: Xiros Limited, Leeds LS19 7UE (GB)
(72) Inventor: SEEDHOM, Bahaa Botros, Leeds Yorkshire LS19 7UE (GB); BEEVERS, David John, Leeds Yorkshire LS19 7UE (GB)
(74) Representative: Orr, Robert
(86) International application number: PCT/GB2013/051317
(87) International publication number: WO 2013/186525

(56) References cited:
- EP-A1- 0 642 773
- EP-A1- 1 334 702
- EP-A1- 1 493 404
- EP-A2- 0 145 492
- US-A- 4 662 886
- US-A1- 2010 292 792

## Description

The present invention relates to synthetic ligament assemblies for implantation in a body of a patient. In particular, but not exclusively, the present invention relates to synthetic ligament assemblies for the reconstruction of the anterior cruciate ligament (ACL). The present invention also relates to methods of implanting a synthetic ligament assembly in a body of a patient.

Ligament damage, including ACL ruptures, are common in many sports. In the past, one way in which reconstruction of an ACL was carried out was by harvesting two hamstring tendons from a patient, and implanting the tendons in a position where they perform the function of the damaged ACL. Harvesting two hamstring tendons from the patient seriously limited the residual hamstring function of the patient. Research has been carried out into synthetic ligaments for use generally in the reconstruction of damaged ligaments in the human body. One particular area in which a significant amount of research has been carried out is in ACL reconstruction. The following is a brief summary of some of the techniques, ligaments and assemblies which have been employed.

Elongate textile structures have been used in ACL reconstruction which attach the knee bones, femur and tibia, where a ruptured ACL remains. The elongate structures are intended to bear tensile loading, and may also function as scaffolds that encourage tissue in-growth. The elongate structures are attached to bone with a fixation device at one end, and anchored to bone with another fixation device at its other end. One such elongate structure is a tubular open weave textile scaffold known as the JewelACL™ ligament, available from the present applicant. The ligament can be suspended at a femoral end within a device known as an ECL, which comprises an anchoring device known as an EndoButton™ and a captive continuous loop (available from Smith & Nephew, Inc.). A tibial end of the ligament can be fixed within a tibial tunnel with an interference screw, or on the external bony surface of the tibia with staples or a similar such device.

In one mode of use, the ligament and fixation components are used alone, thereby obviating the need to use additional tissue for the reconstruction, whether autologous (derived from the subject patient) or allogenic (derived from a genetically non-identical donor). In another mode of use, the ligament and fixation components are used in conjunction with a single hamstring tendon, which can be suspended in the loop of the ECL discussed above, either alongside the ligament or within its tubular structure. In the first mode, the ligament functions as a total tissue sparing graft. In the second mode, the ligament functions as a partial tissue sparing graft, replacing one of the two hamstring tendons that were previously commonly used in the reconstruction of an ACL.

The attached drawing Fig. A illustrates a tubular open weave textile scaffold 1, an anchoring device in the form of an EndoButton™ 2, a captive continuous loop 3 and a graft 4 comprising a harvested hamstring tendon. The graft 4 has been prepared according to standard procedures, such that each end is whip-stitched with sutures 5 to a length of approximately 35 to 40 mm. It has been known to plasma treat the ligament scaffold 1 to render it hydrophilic, improving cell adhesion to its surface and thereby expediting the induction of tissue around and within it, thus forming a neoligament comprising natural tissue and synthetic fibres. One difficulty with this assembly is that the EndoButton™ 2 must be passed along the length of the bone tunnel and then 'flipped' to stride the mouth of the tunnel, and seat against the external surface of the bone. This restricts the size of the EndoButton™ which can be used. A small EndoButton™ exerts a large force on the bone in use, which can cause bone resorption. However, increasing the size of the EndoButton™ to counteract this is undesirable for two reasons. Firstly, a larger EndoButton would necessarily require drilling a tunnel of a larger diameter, which presents its own problems. Secondly, for the EndoButton™ to stride the tunnel effectively, it would have to be made longer. This would make flipping of the device very difficult, or indeed impossible, without making a skin incision above the tunnel, this being a step which surgeons have managed to avoid by using the EndoButton™.

In variations on the above technique, so-called 'toggles' (which are thin, generally dogbone shaped components) have been used to retain synthetic devices at the ends of bony tunnels. One example is the device used for reconstructing an antero-inferior gleno-humeral ligament at the shoulder (Neoligaments/Xiros Limited), disclosed in UK Patent Publication No. GB-2274992.

Anchoring devices, similar to the EndoButton™ of Smith & Nephew described above, have also been used in conjunction with open weave tapes having the weft removed from their central sections, as shown in drawing Fig. B attached. An open weave tape 6 is shown which has been threaded through two holes 7 in an anchoring device 2. In this way, a 'de-wefted' section 8 passes through the holes 7, and two ends 9a, 9b of the tape 6 are then knotted to form a loop (not shown), from which hamstring tendons are suspended for ACL reconstruction. The process of de-wefting the tape 6 reduces the bulk of the material passing within the central holes 7 of the anchoring device 2. The open weave structure of the tape was deemed advantageous in that it encouraged tissue in-growth. However, the size of the device 2 and the small holes 7 was such that the tape 6 used to suspend the tendons needed to be of small width, and therefore possessed much lower strength than that of the tendons used for repair. Accordingly, whilst the tendons themselves possessed considerable strength, the link between them and the bone did not.

Interference fixation screws have been used to jam/trap ligament grafts within bony tunnels. The portion of the tunnel that is not occupied by the screw becomes filled with tissue that bridges the space between the graft/ligament implant and the walls of the tunnels, and with time bears some of the tensile load acting along the implant, which is transmitted onto the screw/implant/bony tunnel interfaces. The longer the portion of the tunnel left for tissue to occupy, the larger the fraction of the tensile load that will be taken by the natural tissue; this is desirable since it is possible that, in the long-term, bone resorption might occur at the tip of the screw within the tunnel, and fretting of the implant against the screw might occur, causing failure of the implant.

International Patent Publication No. WO-01/32229 discloses an implant for tissue repair, particularly the repair of connective tissue such as ligament. The implant comprises a first component in the form of an elongate fabric tape, and a second component in the form of a non-woven felt. The felt is of a material capable of being seeded with and supporting the growth of cells. The felt is attached to the fabric by stitching it on to one of the opposing surfaces of the fabric, and the joined components are then spirally wound to form a structure having a 'swiss-roll' type shape. While research has shown evidence of tissue in-growth between the fabric/felt layers, the layers are closely compact and the fabric densely woven, with the result that the in-grown tissue does not have continuity along the axial direction of the device, and so cannot transmit load. Document US4662886 also discloses a woven synthetic ligament assembly. As can be understood from the above text, a variety of components and design features have been used in combination for ACL (and other ligament) reconstruction. Whilst at least some of these have proved to be reasonably effective, it is desired to improve upon the respective structures and methods employed.

According to a first aspect of the present invention, there is provided a synthetic ligament assembly comprising:
an elongate woven ligament scaffold which can be located in a tunnel in a bone, the scaffold comprising a plurality of warps extending in a length direction of the scaffold and a plurality of wefts extending transverse to the warps, a region of the scaffold between opposed ends thereof being free from wefts, and the scaffold being shaped so that it defines an internal space;
an inner component which is treated to promote the in-growth of tissue following implantation, the inner component located within the internal space defined by the scaffold; and
a toggle having a bone facing surface;
in which the scaffold is folded in the weftless region to form an eye which receives the toggle so that warps in the weftless region of the scaffold pass around part of a perimeter of the toggle, the scaffold and the inner component being securable in the bone tunnel by contact between the bone facing surface of the toggle and a surface of the bone. The inner component may be received in the internal space of the scaffold in such a way that relative movement between the inner component and the scaffold is permitted (that is, without an additional mechanical bond between the inner component and the scaffold). In this way, the assembled structure comprising the scaffold and the inner component may be an open one, which promotes the in-growth of tissue. This is in contrast to prior structures such as that disclosed in WO-01/32229, in which the joining of the disclosed first and second components together, and the spiral winding, forms a structure which is relatively dense and compact, and which does not promote in-growth of effective/functional tissue, i.e. tissue which is capable of transmitting load. The inner component may be received in the internal space in the scaffold in such a way that free movement of the inner component is possible, bounded or restricted only by a wall or walls of the scaffold.

The scaffold, which is the main load bearing component of the assembly, may be generally tubular. This may be achieved by folding an elongate woven fabric sheet about a main longitudinal axis and securing longitudinal edges of the fabric sheet together. It can also be woven as a unitary structure. It will be understood that the fabric sheet is flexible/collapsible, and so references to the scaffold being tubular should not be taken to imply that the scaffold has a fixed shape in cross-section (e.g. circular).

The internal space in the scaffold may be a passage extending in a length direction of the scaffold. The passage may extend along an entire length of the scaffold. The passage may extend partway along a length of the scaffold. The assembly may comprise a plurality of inner components. The inner component may be an elongate strip. The scaffold may comprise at least one opening/slit extending in a length direction of the scaffold, the opening serving for inserting the inner component into the scaffold. The scaffold may comprise at least one pocket extending partway along a length of the scaffold, which pocket may communicate with said opening.

The assembly may comprise a draw or pull cord for pulling the scaffold and the inner component through the bone tunnel. The weftless region of the scaffold may be provided at a midpoint along a length of the scaffold such that, when folded, the opposed ends of the scaffold meet, and the draw cord may be attached to said ends.

The toggle may be inserted in the eye and the scaffold secured relative to the toggle so as to prevent, or at least restrict, removal of the toggle from the eye. The toggle may be secured by means of a whipping extending around the scaffold transverse to a main axis of the scaffold, which may be positioned proximate to the toggle and which may extend around warps in the weftless region of the scaffold. When applied, the whipping reduces the effective dimension of the eye so that the toggle cannot be removed. The whipping may comprise a thread, cord or the like and may be of the same material as the scaffold. The toggle may be secured by means of a sleeve positioned around the scaffold. The sleeve may be generally tubular. The sleeve may be densely woven. The sleeve may be of a weave having a greater density than that of the scaffold. The sleeve may be of a weave having a greater density than at least a majority or main part of the scaffold (excluding for example end regions of the scaffold, which may be of greater density than a remainder of the scaffold for receiving a draw cord). The sleeve may be of a biocompatible yarn, and may be of a yarn which is the same as that of the scaffold. The sleeve may be secured to itself through the scaffold. The sleeve may be secured to the scaffold at or adjacent its end furthest from the toggle. The sleeve may be secured to the scaffold at or adjacent its end closest to the toggle. The sleeve may be secured to the toggle, such as via yarns or stitches extending around the toggle, or secured to the warps in the weftless region. The sleeve may be secured with stitches, which may be of a similar yarn as that of the scaffold. The sleeve may retain the toggle and prevent it from being dislodged or rotated. The sleeve may protect the ligament scaffold from abrasion against the entrance of the tunnel where the toggle is attached; such tunnels might have sharp edges that can damage the ligament scaffold.

The inner component may be of a non-woven material, and may be a needle-punched material (felt). The inner component may be subjected to a treatment process which makes it hydrophilic, to thereby promote tissue in-growth. The inner component may be plasma treated. The inner component may be of a plastics material, and may be of polyethylene terephthalate. The scaffold may be of the same material as the inner component. The inner component may have a high porosity, and may have a porosity of at least 90%. This may promote the in-growth of tissue.

The above assembly is intended to be used alone for reconstruction of a ligament (particularly the ACL), without any additional tissue (thus functioning as a total tissue sparing device). It will therefore typically be supplied with the toggle already attached to the scaffold, which is whipped as described to retain the toggle in place; with the non-woven inner component already inserted within the space of the (optionally) tubular structure of the woven scaffold, and with the draw cord attached to the end of the woven scaffold. Said end of the woven scaffold may be made of a dense weave so as to provide a robust attachment for the draw cord. According to a second aspect not being part of the present invention, there is provided a synthetic ligament assembly comprising:
an elongate woven ligament scaffold which can be located in a tunnel in a bone, the scaffold comprising a plurality of warps extending in a length direction of the scaffold and a plurality of wefts extending transverse to the warps, in which a region of the scaffold between opposed ends thereof is free from wefts;
a support for harvested tissue which can be located at least partly in the bone tunnel, the support formed into a loop which can receive and support the tissue; and
a toggle for receiving the scaffold and the support, the toggle having a bone facing surface for securing the scaffold and the support within the bone tunnel;
in which the scaffold is folded in the weftless region and secured to the support to form an eye comprising portions of the warps in the weftless region of the scaffold and part of the support, the eye dimensioned to receive the toggle so that, in use, the scaffold and the support can be secured in the bone tunnel by contact between the bone facing surface of the toggle and a surface of the bone.

The eye may be dimensioned so that the toggle can be inserted into the eye following location of the scaffold and the support in the bone tunnel. The eye may therefore describe a maximum dimension which is greater than a maximum width dimension of the toggle. The support may define a continuous, optionally knotless, loop. The scaffold may be secured/securable to the support by a whipping extending around the scaffold transverse to a main axis of the scaffold. The whipping may additionally/alternatively secure the toggle within the eye. The scaffold may be secured/securable to the support by means of a sleeve positioned around the scaffold. The sleeve may be generally tubular. The sleeve may additionally/alternatively secure the toggle in the eye. The sleeve may be initially positioned around the scaffold such that the sleeve can move relative to the scaffold. The sleeve may be movable between an initial position in which insertion of the toggle into the eye is permitted, and a restraining position in which the sleeve prevents release of the toggle from the eye. The sleeve may therefore secure the toggle within the eye. In this aspect of the invention, the step of securing the toggle in the eye may be carried out during a surgical procedure to implant the assembly, following positioning of the toggle in the eye.

The sleeve may be densely woven. The sleeve may be of a weave having a greater density than that of the scaffold. The sleeve may be of a weave having a greater density than at least a majority or main part of the scaffold (excluding for example end regions of the scaffold, which may be of greater density than a remainder of the scaffold for receiving a draw cord). The sleeve may be of a biocompatible yarn, and may be of a yarn which is the same as that of the scaffold. The sleeve may be secured to itself and to the support through the scaffold. The sleeve may be secured to the scaffold and to the support at its end furthest from the toggle. The sleeve may be secured to the scaffold and to the support at its end closest to the toggle. The sleeve may be secured with stitches, which may be of a similar yarn as that of the scaffold. The tissue may, for example, comprise a tendon which can be passed through the loop defined by the support and so secured in the bone tunnel. The assembly may comprise a draw cord for pulling the scaffold and the support through the bone tunnel. The draw cord may be attached to the eye, and may be removed prior to insertion of the toggle in the eye.

As mentioned the above assembly is intended to be used in conjunction with tissue for ligament reconstruction, and may be intended to be used with one hamstring tendon for ACL reconstruction (thus as a partial tissue sparing device). It may therefore be supplied with the scaffold folded at its weftless section over the loop, and whipped to retain the loop securely. The cord may suspend the scaffold and loop at the end that is adjacent to the whipping/sleeve. The eye or eyelet from which the cord suspends the scaffold and loop will be sufficient to introduce a toggle during surgery.

Reference is made herein to a scaffold comprising a plurality of warps and wefts. It will be understood that the terms 'woof and 'filler' may be used interchangeably with the term 'weft'. Typically the scaffold will comprise a plurality of threads forming the warps, whilst a single weft thread may define a plurality of wefts. It will be understood that the scaffold will typically comprise at least two weft threads, each weft thread forming, together with the warp threads, portions of the scaffold either side of the weftless region.

The toggle may comprise first and second ends and a waist portion positioned between the ends, the waist portion being of a smaller width than that of the first and second ends. Reference to the width of the waist portion and first and second ends should be taken to be in a direction which is in the same plane as the bone facing surface defined by the toggle, and perpendicular to a direction of insertion of the toggle into the eye. The waist portion may be shaped to receive the weftless region of the scaffold. Forming the scaffold to have the weftless region may provide the advantage of reducing a width of the scaffold and the volume of material which passes around the toggle waist.

The first and second ends of the toggle may comprise inner surfaces which together define the bone facing surface of the toggle. The first and second ends may comprise outer surfaces, opposite to the respective inner surfaces, and the outer surfaces may be arcuate. This may provide the advantage of avoiding the formation of a hard ledge or edge, which may cause discomfort for the patient. The first and second ends may be rounded or domed. The outer surfaces of the first and second ends may curve in the more than one plane.

The waist portion of the toggle may be of a height which is less than that of the first and second ends. This may provide a recess in which the weftless region of the scaffold can sit when the toggle is positioned in the eye, resisting removal of the toggle from the eye and reducing the profile of the assembly. The toggle may comprise a locating element which extends from the bone facing surface which, in use, locates in an opening of the bone tunnel. The locating element may define an abutment surface which is shaped to abut an internal wall of the bone tunnel. The locating element may taper in a direction away from the bone facing surface. The abutment surface may be disposed at a non-parallel angle to a normal of the bone facing surface. The abutment surface may be disposed at an angle of between about 1° to about 15° relative to the normal. The locating element may be elongate and may be oriented so that its length dimension is parallel to a main axis of the toggle. The locating element may be of a dimension (which may be a length where it is elongate) which is selected so that the element centres the toggle relative to the opening of the bone tunnel. This may provide a good contact between the inner surfaces of the first and second ends of the toggle and the surface of the bone. Said dimension may be slightly less than the diameter of the tunnel opening, and may be in the region of 95% to 99% of the diameter.

According to a third aspect not being part of the present invention, there is provided a toggle for receiving a synthetic ligament to position the ligament within a tunnel in a bone, the toggle comprising:
first and second ends;
a waist portion positioned between the ends, the waist portion being of a width which is smaller than that of the first and second ends and of a height which is smaller than that of the first and second ends;
a bone facing surface which can contact a surface of a bone to position a ligament coupled to the toggle within a bone tunnel; and
a locating element which extends from the bone facing surface;
in which, in use, the ligament extends around part of a perimeter of the waist portion of the toggle, and the locating element is located in an opening of the bone tunnel and dimensioned so that it centres the toggle relative to the opening.

Embodiments of the present invention will now be described, with reference to the accompanying drawings, in which:
Fig. A is a front view of a synthetic ligament assembly of a type known in the art and comprising an open weave textile scaffold, anchoring device, captive continuous loop and a hamstring tendon graft;
Fig. B is a view of a synthetic ligament attachment assembly of a type known in the art and comprising an open weave tape and anchoring device;
Fig. 1 is a schematic front view of a joint between a femur and a tibia of a patient who has suffered ACL rupture, showing the femur and tibia following formation of tunnels in the bones, in preparation for receiving a synthetic ligament assembly according to an embodiment of the present invention;
Fig. 2 is a perspective view of part of a synthetic ligament assembly in accordance with an embodiment of the present invention, to be located in the bone tunnels formed in the femur and the tibia shown in Fig. 1, the assembly having a utility in a total tissue sparing procedure;
Fig. 2a is an enlarged view of part of the ligament assembly shown in Fig. 2, illustrating a scaffold of the assembly, and showing an alternative means for securing a toggle of the assembly to a weft free portion of the scaffold;
Fig. 2b is a view of the means for securing the toggle shown in Fig. 2a, which takes the form of a sleeve;
Fig. 2c is a further enlarged view similar to Fig. 2a;
Fig. 3 is a plan view of a further part of the assembly shown in Fig. 2;
Fig. 4 is a side view of the part of the assembly shown in Fig. 3;
Fig. 5 is a view of the joint between the femur and tibia shown in Fig. 1, shown following location of the synthetic ligament assembly of Fig. 2 in the bone tunnels in the femur and the tibia and anchoring of the assembly in the tunnels;
Fig. 6 is a view illustrating a step in a method of implanting the synthetic ligament assembly of Fig. 2 in the joint of Fig. 1;
Figs. 7 and 8 are enlarged side and perspective views, respectively, of a toggle forming part of the assembly of Fig. 2;
Fig. 9 is a perspective view of a synthetic ligament assembly in accordance with another embodiment of the present invention, to be located in the bone tunnels formed in the femur and the tibia shown in Fig. 1, the assembly having a utility in a partial tissue sparing procedure;
Fig. 10 is a plan view of a further part of the assembly shown in Fig. 9; and
Fig. 11 is a schematic front view of another joint between a femur and a tibia of a patient who has suffered ACL rupture, showing the femur and tibia following formation of tunnels in the bones, in preparation for receiving the synthetic ligament assembly of Fig. 9.

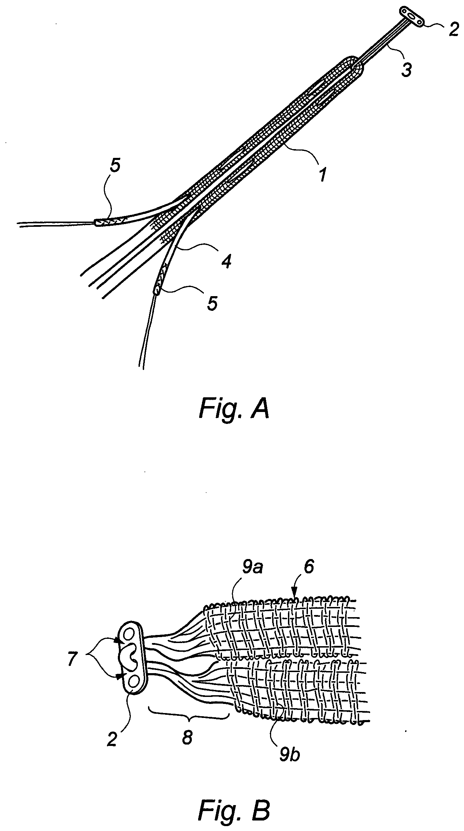

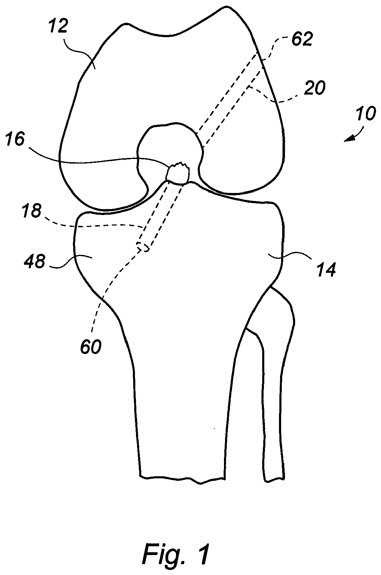

Turning firstly to Fig. 1, there is shown a schematic front view illustrating a joint 10 between a femur 12 and a tibia 14 of a patient who has suffered an ACL rupture. A remnant portion of the natural ACL attached to the tibia 14 is shown in the drawing, and given the reference numeral 16. The joint 10 is shown following the formation of a bone tunnel 18 in the tibia 14 and a bone tunnel 20 in the femur 12, the bone tunnels formed following standard procedures which are known in the art. Formation of the bone tunnels 18 and 20 is a preparatory step to an ACL reconstruction technique employing synthetic ligament assemblies of the present invention, which will now be described.

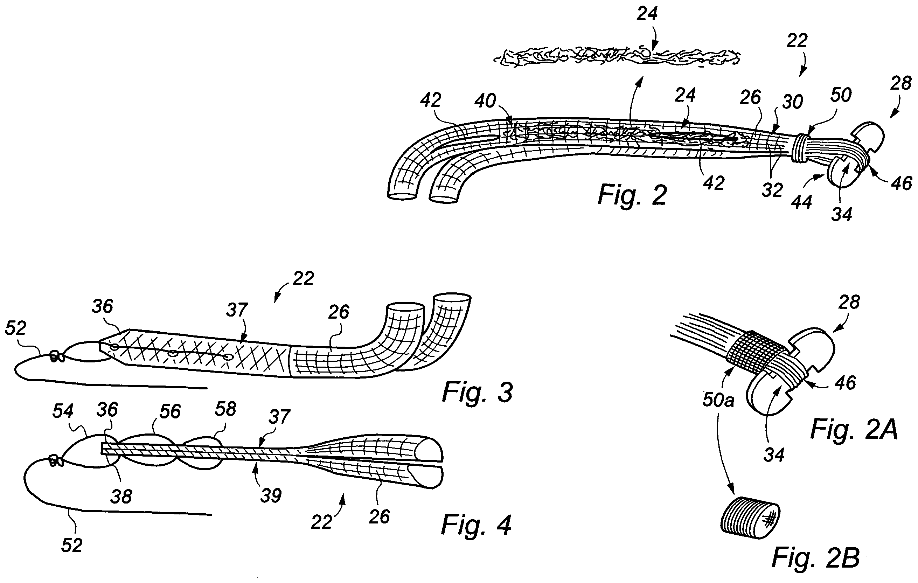

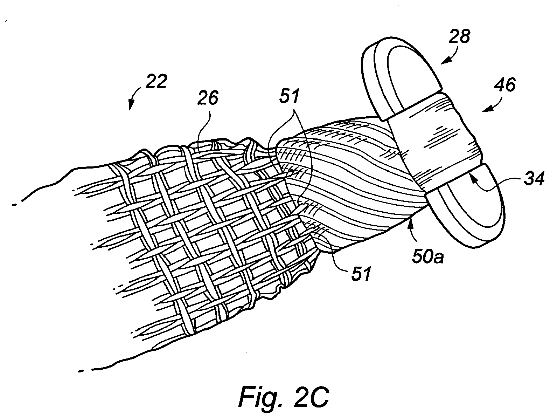

Thus turning to Fig. 2, there is shown a perspective view of a synthetic ligament assembly in accordance with an embodiment of the present invention, the assembly indicated generally by reference numeral 22. The assembly 22 is to be located in the bone tunnels 18 and 20 formed in the tibia 14 and the femur 12, and has a utility in a total tissue sparing procedure. This is appropriate for acute cases, in which the ligament will be used alone (i.e. no autologous or allograft tissues will be incorporated in the reconstruction). The assembly 22 generally comprises an inner component 24 which is treated to promote the in-growth of tissue following implantation, an elongate woven ligament scaffold 26 and a toggle 28. The scaffold 26 can be located in the tunnels 18 and 20 in the tibia 14 and femur 12, and comprises a plurality of warps 30 extending in a length direction of the scaffold 26, and a plurality of wefts 32 extending transverse to the warps 30. As will be understood by persons skilled in the art, the scaffold 26 typically comprises a plurality of warp threads forming the warps 30, with a weft thread passing back and forth across the woven structure and defining a number of the wefts 32. The assembly 22 is also shown in Fig. 3, which is a plan view of a further part of the assembly, and Fig. 4, which is a side view of the part of the assembly shown in Fig. 3.

A region 34 of the scaffold 26 between opposed ends 36 and 38 is free from wefts 32, as best shown in Fig. 2. The region 34 is typically around 30mm in length. The scaffold 26 is shaped so that it defines an internal space 40 which can receive the inner component 24. The internal space 40 can extend along an entire length or a majority of the scaffold 26 (which may then be generally tubular along all or a majority of its length). The scaffold 26 includes an opening 42 which extends in a length direction of the scaffold. During manufacture, the inner component 24 is inserted into the internal space 40 through the opening 42. The internal space 40 can thus be a pocket formed in the scaffold 26. Two elongate strips of the inner component 24 are inserted into the space 40 in the scaffold 26, either side of the de-wefted region 34.

The toggle 28 defines a bone facing surface 44, and the scaffold 26 is folded in the weftless region 34 to form an eye 46 which receives the toggle 28, so that the warps 30 in the weftless region 34 pass around part of a perimeter of the toggle. As will be described in more detail below, the scaffold 26 and the inner component 24 are securable in the bone tunnels 18 and 20 by contact between the bone facing surface 44 of the toggle 28 and a surface 48 of the bone. In the illustrated embodiment, a tibial fixation procedure is employed in which the bone facing surface 44 of the toggle 28 is seated against a surface 48 of the tibia 14. However, and as will be understood by persons skilled in the art, a femoral fixation procedure may be employed (in particular, where the femoral tunnel is short), in which situation the bone facing surface 44 of the toggle 28 would be supported against a surface of the femur 12.

The assembly 22 is provided with the toggle 28 inserted into the eye 46 defined by the scaffold 26. The toggle is secured by a whipping 50 comprising a thread or threads which pass around the scaffold 26 in a direction transverse to its main axis. This prevents, or at least restricts, removal of the toggle 28 from the eye 46.

In a variation shown in Figs. 2a to 2c, an alternative to the whipping 50 is illustrated. This is a short sleeve 50a, densely woven from a biocompatible yarn. The sleeve 50a is of a weave having a greater density than at least a majority or main part of the scaffold 26, which is that part of the scaffold densely woven end sections 37 and 39 of the scaffold 26 (which will be discussed below). The sleeve 50a is typically around 8-12 mm long, and of an inner diameter of typically around 6-9 mm. The sleeve 50a is secured to itself through the scaffold 26 at its end furthest from the toggle with stitches 51 (Fig. 2c) of a similar yarn as the scaffold 26. The sleeve 50a may also be secured to the toggle 28, such as via yarns or stitches (not shown) extending around the toggle 28, or secured to the warps 30 in the weftless region 34. The function of the sleeve 50a is twofold. The first is to retain the toggle 28 and prevent it from being dislodged or rotated. The second is to protect the ligament scaffold 26 from abrasion against the entrance of the tunnel 18 where the toggle 28 is attached; such tunnels might have sharp edges that can damage the ligament scaffold 26.

A draw cord 52 is secured to the ends 36 and 38 of the scaffold 26, as best shown in Figs. 3 and 4. The draw cord 52 may be threaded a number of times through the densely woven end sections 37 and 39 of the scaffold 26 to form loops 54, 56 and 58, for added strength. The inner component 24 is inserted into the scaffold 26 during manufacture, and so supplied pre-installed to the surgeon. The inner component 24, scaffold 26 and whipping 50/sleeve 50a are typically of the same material. A plastics material, particularly polyethylene terephthalate (commonly known by a brand name such as polyester) is preferred, although other materials may be suitable. The inner component 24 is typically provided as an elongate strip of a non-woven material, and may be a needle-punched material (felt). The inner component 24 is subjected to plasma treatment, to enhance its surface properties and make the inner component hydrophilic, so that cells can readily adhere to it, to expedite a process of tissue induction that spreads to populate the structure of the scaffold 26.

Although both the ligament scaffold 26 and the non-woven strips forming the inner components 24 are made from the same material (preferably polyethylene terephthalate), only said non-woven strip(s) 24 is subjected to plasma treatment. This enhances its surface properties so that cells can readily adhere to it, and so expedites a process of tissue induction that spreads to populate the structure of the scaffold 26. For the tissue induced to be effective in bearing load, structures of both the scaffold 26 and the non-woven strip 24 should ideally possess loose structures, with the bulk of their material occupying a small fraction of the structure volume, thus leaving ample space for tissue to grow and occupy this space. The open weave structure of the scaffold 26 and the loose structures of the non-woven strips 24 (which typically have an equivalent porosity of 90% or more) are highly appropriate for this process of tissue induction. The inner component 24 is located within the space 40 of the scaffold 26 loosely, and is not secured to the scaffold such as by stitching. The inner component 24 is thus movable relative to the scaffold 26, contained or bounded only by a wall or walls of the scaffold. This helps to maintain the open structure and promotes tissue in-growth.

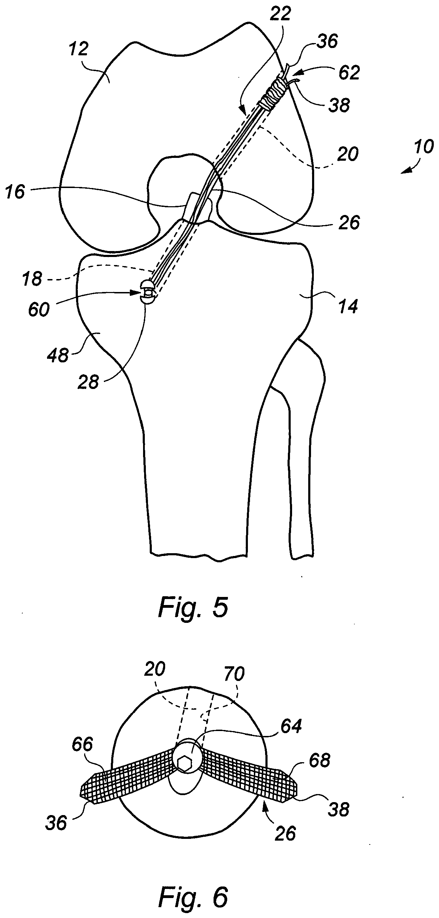

The assembly 22 can be inserted into the joint 10 for the reconstruction of the ACL without any additional tissue. This is achieved by firstly inserting draw cord 52 into an entrance hole 60 of the tibial tunnel 18, and then threading the draw cord through the tibial tunnel 18, up through the femoral tunnel 20, and out of an exit hole 62 of the femoral tunnel. The draw cord 52 is then pulled up through the bone tunnels 18 and 20 carrying the scaffold 26, and drawn up until the toggle 28 rests with its flat, bone facing surface 44 on the surface 48 of the tibia 14, striding the bone either side of the tibial tunnel 60, as shown in Fig. 5. Tension can then be applied to the scaffold 26, and the scaffold anchored to the femur 12, as shown in Fig. 5. Anchoring to the femur is achieved using an interference screw 64, which is shown in the enlarged view of Fig. 6 (although other suitable means such as a staple may be employed). The interference screw 64 clamps portions 66 and 68 of the scaffold 26 against an internal wall 70 of the femoral tunnel 20. Positioning the screw 64 in the femoral tunnel 20 is advantageous because the femoral tunnels will usually be longer than the tibial tunnel 18, and so provides a greater portion of open bone surrounding the scaffold 26. In addition, it has been found that bone material of the femur 12 is usually stronger than that of the tibia 14, providing a more secure anchoring and reducing the likelihood of bone resorption.

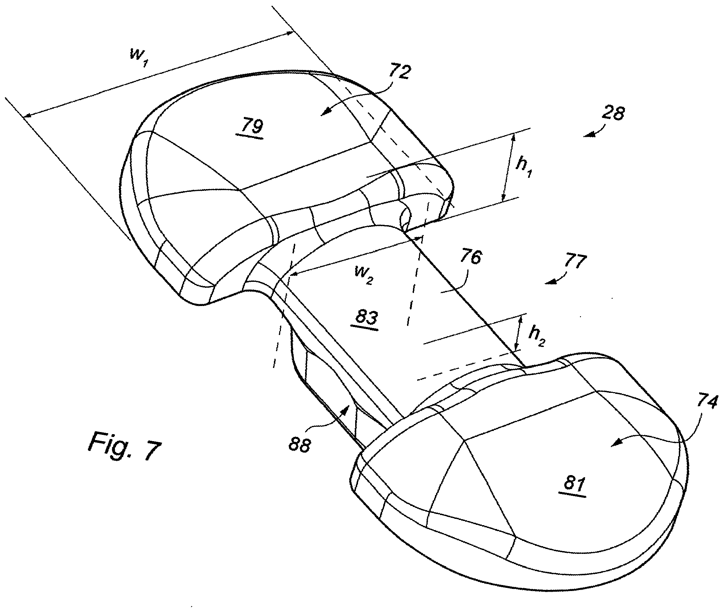

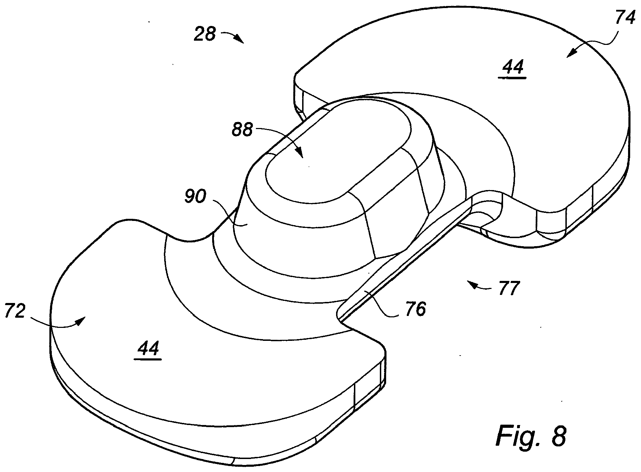

The toggle 28 is shown in more detail in the enlarged perspective views of Figs. 7 and 8, taken respectively from above and below. The toggle 28 is typically of a biocompatible grade titanium material and may be manufactured under a laser sintering or other suitable process. The toggle 28 comprises first and second ends 72, 74 and a waist portion 76. The ends 72 and 74 are of larger width w₁ than a width w₂ of the waist portion 76. In this way, an area 77 is defined which can receive the warps 30 of the weftless region 34 of the scaffold 26, the warps passing around part of a perimeter of the toggle 28. The toggle 28 is thus retained in the eyelet 46 following insertion, as shown in Fig. 2. The ends 72 and 74 are also of larger height h₁ than a height h₂ of the waist portion 76. In this way, the area 77 is effectively recessed relative to the ends 72 and 74, so that the warps 30 of the weftless region 34 do not protrude (or protrude to a lesser extent than would otherwise be the case) beyond upper surfaces 79 and 81 of the ends 72 and 74. The ends 72 and 74 are also curved in two planes, and so generally domed, so that they provide a minimum upset, reducing discomfort for the patient following implantation. An upper surface 83 of the waist portion 76 is also curved.

The toggle 28 also comprises a locating element 88 which extends from the bone facing surface 44. In use, the locating element 88 is positioned in the opening 60 of the tibial bone tunnel 18. The locating element 88 defines a lateral abutment surface 90 which is shaped to abut an internal wall of the tibial bone tunnel 18. The locating element 88 tapers in a direction away from the bone facing surface 44, and may be disposed at a non-parallel angle to a normal extending from the bone facing surface, typically at an angle of between about 1° to about 15° relative to the normal. The locating element 88 is elongate, and oriented so that its length dimension is parallel to a main axis of the toggle 28. The locating element 88 is of a dimension which is selected so that the element 88 centres the toggle 28 relative to the opening 60 of the tibial bone tunnel 18; in the illustrated embodiment, said dimension is the length of the element 88. This ensures that there is a good contact between the inner surfaces of the first and second ends 72 and 74 of the toggle (which define the bone facing surface 44) and the surface 48 of the tibial bone 14. The dimension is slightly less than the diameter of the tunnel opening 60, and is typically in the region of 95% to 99% of the diameter, so as to best centre the toggle 28 relative to the opening.

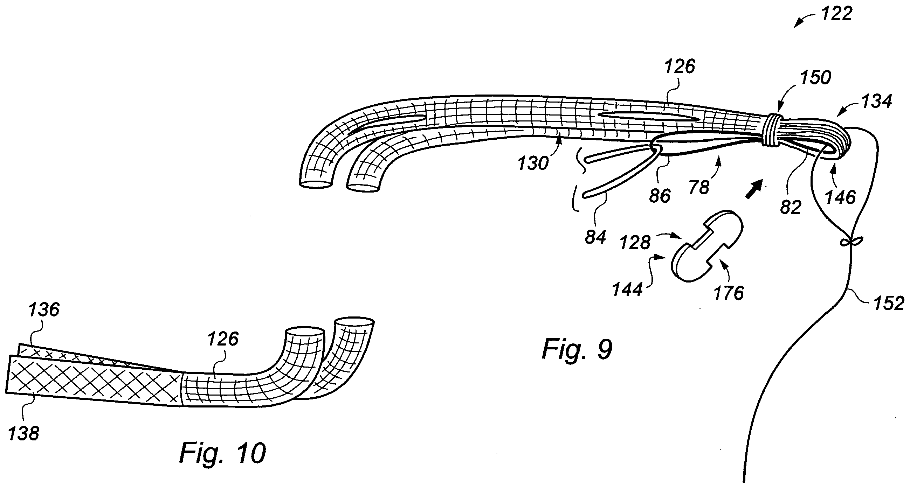

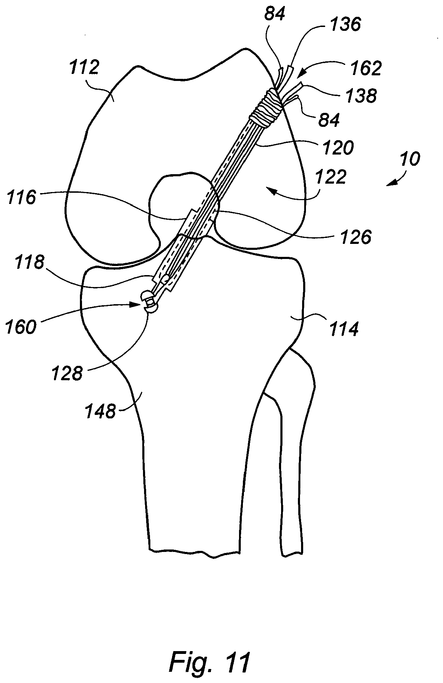

Turning now to Fig. 9, there is shown a perspective view of a synthetic ligament assembly, the assembly indicated generally by reference numeral 122. Part of the assembly 122 is also shown in the plan view of Fig. 10. Like components of the assembly 122 with the assembly 22 of Figs. 2 to 8 share the same reference numerals, incremented by 100. The assembly 122 has a utility in a partial tissue sparing procedure, and is located in bone tunnels of a joint which are similar to the tunnels 18 and 20 of the joint 10 shown in Fig. 1. The assembly 122 is shown located in bone tunnels 118 and 120 of such a joint 100 in Fig. 11. The assembly 122 is appropriate for use in chronic cases, in which the ligament will be used in conjunction with harvested tissue. As will be described, in this embodiment, the bone tunnel 118 in the tibia 114 is stepped, being of a smaller diameter proximal to the tibial tunnel hole 160, as shown in Fig. 11.

In common with the assembly 22 of Fig. 2, the assembly 122 comprises a scaffold 126 having a weftless region 134 and a toggle 128. However, the toggle 128 is inserted following positioning of the scaffold 126 in the bone tunnels 118, 120. The assembly 122 includes a support for harvested tissue, the support formed into a loop and indicated generally by reference numeral 78. In the case of an ACL reconstruction procedure, the harvested tissue will typically be a hamstring tendon 84 harvested from the patient or from a donor.

In a similar fashion to the scaffold 26, the scaffold 126 is folded in the weftless region 134. The scaffold 126 is then secured to the support 78 to form an eye or eyelet 146 comprising portions of warps 130 of the scaffold 126, and a part 82 of the support 78. The support 78 is secured to the scaffold 126 to form the eyelet 146 using a whipping, as shown at 150 in the drawing, or by other suitable means. Again however, the scaffold 126 may optionally be secured to the support 78 by means of a sleeve positioned around the scaffold, such as the sleeve 50a shown in Fig. 2a and described above. The eye 146 is dimensioned to receive the toggle 128 so that, in use, the scaffold 126 and the support 78 can be secured in the bone tunnels 118 and 120 by contact between a bone facing surface 144 of the toggle 128 and the surface 148 of the tibia 114. Again, it will be understood that the assembly 122 may be anchored relative to the femur 112, if desired.

As mentioned above, a hamstring tendon numeral 84 is introduced into an eye 86 which is formed following whipping of the support 78 to the scaffold 126 (or positioning of the sleeve where used instead of the whipping). The hamstring tendon 84 is thus secured to the assembly 122, which is then ready for implantation. Incorporation of the hamstring tendon 84 results in the assembled implant 122 being of a larger overall width dimension in the region of the hamstring tendon than in the portion of the assembly between the tendon and the eye 146. As a result, the bone tunnel 118 in the tibia 114 may have to be stepped as shown, so that it is of a larger diameter in the area which is to receive the tendon. This will depend upon the combined width of the assembly 122 and the tendon 84. Known techniques can be employed to form a stepped tunnel. This is advantageous not only in reducing trauma to the patient, but also in that it provides a greater contact area with the bone facing surface 144 of the toggle 128, reducing pressure on the bone surface 148 and so the likelihood of bone resorption.

A draw cord 152 is secured to the scaffold 126 and support 78 through the eye 146. The draw cord 152 is then fed into the hole 162 in the femur 112, which in this instance forms an entrance hole. The draw cord 152 is fed on up through the femoral tunnel 120 and into the tibial tunnel 118, exiting the hole 160, which in this instance forms an exit hole. The draw cord 152 is then pulled through the bone tunnels 118 and 120, and the eye 146 fed into the entrance hole 162 of the femoral tunnel 120. The joined scaffold 126, support 78 and coupled hamstring tendon 84 are thus fed up through the bone tunnels. This continues until the eye 146 protrudes from the exit hole 160 of the tibial tunnel 118. The draw cord 152 can then be removed, and the toggle 128 inserted through the eye 146. The warps 130 in the weftless region 134 of the scaffold 126 are positioned so that they pass around a waist portion 176 of the toggle 128. Ends 136 and 138 of the scaffold 126 are then pulled in a direction back down through the bone tunnels 118 and 120, to bring the bone facing surface 144 of the toggle 128 into contact with the surface 148 of the tibia 114. The scaffold 126 can then be anchored in a similar fashion to the scaffold 26, employing an interference screw (not shown). In this instance, the interference screw also serves for anchoring the hamstring tendon 84.

The whipping 150 may be applied to the scaffold 126 following insertion of the toggle 128 in the eye 146, to secure the toggle in the eye and prevent toggle release. Where a sleeve (such as the sleeve 50a) is used in place of the whipping 150, the sleeve may be initially positioned around the scaffold 126 such that it can move relative to the scaffold. The sleeve may be movable between an initial position in which insertion of the toggle 28 into the eye 146 is permitted, and a restraining position (as shown in Figs. 2a and 2c) in which the sleeve prevents release of the toggle 128 from the eye 146. The sleeve may therefore secure the toggle 128 within the eye 34. In this embodiment of the invention, the step of securing the toggle 128 in the eye 146 will be carried out during the procedure to implant the assembly 122, following positioning of the toggle in the eye. A means other than the sleeve may be employed to secure scaffold 126 to the support 78 to facilitate such movement of the sleeve.

In the invention described above, a portion of the natural ACL may remain, as indicated by reference 16 in Fig. 1. This can be sutured to the scaffold 26, 126 to either: act as a source of cell material for populating the scaffold 26 and inner component 24; or to form a composite ligament structure with the scaffold 126 and hamstring tendon 84. Frequently, a rupture of the ACL will occur proximate to the femur 12, and feeding the total tissue sparing synthetic ligament assembly 22 up through the tibial bone tunnel 18 into the femoral bone tunnel 20 can assist in preventing bunching of the ACL remnant 16, and also dragging the remnant towards the femoral bone tunnel 20. This can assist suturing of the ACL remnant 16 to the scaffold 126. In the case of the partial tissue sparing synthetic ligament assembly 122, the assembly is fed from the femoral tunnel 120 into the tibial tunnel 120, to prevent bunching of the hamstring tendon 84.

The synthetic ligament assemblies of the present invention offer numerous advantages over the prior assemblies discussed above. Advantages include the following.

A toggle appropriate for the above applications would provide a much larger area of contact with bone than in prior assemblies such as those incorporating an EndoButton™, which may only provide a contact area of around 28 to 30 mm². That provided by a toggle may be around 65 mm² or more, which considerably reduces the contact stresses with bone, more than halving it. This is important as bone can resorb under the effect of high stress concentrations. Also, a toggle with a large area of contact is less likely to sink into the bone than an EndoButton™ with half its area of contact.

Because of its design, the toggle allows the scaffold to be suspended directly to it, rather than through a support such as a continuous loop, which has a lower strength than the scaffold. It thus reduces the number of components required for the ACL reconstruction where this reconstruction does not require the use of tissue.

A toggle does not occupy any portion of a bony tunnel, thus allowing the whole of the tunnel to be made available for tissue in-growth, thereby achieving a biological fixation of the scaffold within the tunnel. This is much more desirable than fixation that is achieved with an interference screw that largely occupies a short bone tunnel, a situation that is frequently encountered at the tibia.

A toggle saves theatre time as, where it is used, the step of introducing an interference screw is eliminated.

The toggle allows the incorporation of a continuous loop to suspend a hamstring tendon.

## Claims

1. A synthetic ligament assembly (22) comprising:
an elongate woven ligament scaffold (26) which can be located in a tunnel (18, 20) in a bone (12, 14), the scaffold comprising a plurality of warps (30) extending in a length direction of the scaffold and a plurality of wefts (32) extending transverse to the warps, a region (34) of the scaffold between opposed ends (36, 38) thereof being free from wefts, and the scaffold being shaped so that it defines an internal space (40);
an inner component (24) which is treated to promote the in-growth of tissue following implantation, the inner component located within the internal space defined by the scaffold; and
a toggle (28) having a bone facing surface (44);
in which the scaffold is folded in the weftless region to form an eye (46) which receives the toggle so that warps in the weftless region of the scaffold pass around part of a perimeter of the toggle, the scaffold and the inner component being securable in the bone tunnel by contact between the bone facing surface of the toggle and a surface of the bone.

2. An assembly as claimed in claim 1, in which the inner component is located in the internal space of the scaffold in such a way that relative movement between the inner component and the scaffold is permitted.

3. An assembly as claimed in claim 2, in which the inner component is located in the internal space in the scaffold in such a way that free movement of the inner component is possible, restricted only by a wall or walls of the scaffold.

4. An assembly as claimed in any preceding claim, in which the scaffold, which is the main load bearing component of the assembly, is generally tubular.

5. An assembly as claimed in any preceding claim, comprising a draw cord (52) for pulling the scaffold and the inner component through the bone tunnel.

6. An assembly as claimed in any preceding claim, in which the toggle is inserted in the eye and the scaffold secured relative to the toggle so as to prevent removal of the toggle from the eye.

7. An assembly as claimed in claim 6, in which the scaffold is secured by means of a whipping (50) extending around the scaffold transverse to a main axis of the scaffold, the whipping positioned proximate to the toggle.

8. An assembly as claimed in claim 6, in which the toggle is secured by means of a sleeve (50a) positioned around the scaffold, the sleeve being of a weave having a greater density than at least a main part of the scaffold.

9. An assembly as claimed in any preceding claim, in which the inner component is of a non-woven material and is subjected to a treatment process which makes it hydrophilic, to thereby promote tissue in-growth.

10. An assembly as claimed in any preceding claim, in which the toggle comprises first and second ends (72, 74) and a waist portion (76) positioned between the ends, the waist portion being of a smaller width (w₂) than that of the first and second ends (w₁) so that it can receive the weftless region of the scaffold.

11. An assembly as claimed in claim 10, in which:
the first and second ends of the toggle comprise inner surfaces which together define the bone facing surface of the toggle;
the first and second ends of the toggle comprise outer surfaces (79, 81), opposite to the respective inner surfaces; and
the outer surfaces are arcuate.

12. An assembly as claimed in claim 11, in which the outer surfaces of the first and second ends curve in more than one plane.

13. An assembly as claimed in any one of claims 10 to 12, in which the waist portion of the toggle is of a height (h₂) which is less than that of the first and second ends (h₁) to define a recess (77) in which the weftless region of the scaffold can sit when the toggle is positioned in the eye, resisting removal of the toggle from the eye and reducing the profile of the assembly.

14. An assembly as claimed in any one of claims 10 to 13, in which the toggle comprises a locating element (88) which extends from the bone facing surface and which, in use, locates in an opening (60) of the bone tunnel.

15. An assembly as claimed in claim 14, in which the locating element defines an abutment surface (90) which is shaped to abut an internal wall of the bone tunnel, and in which the locating element tapers in a direction away from the bone facing surface of the toggle.

## Patentansprüche

1. Synthetische Ligamentanordnung (22), umfassend
ein langgestrecktes, gewobenes Ligamentgerüst (26), welches in einem Kanal (18, 20) in einem Knochen (12, 14) angeordnet sein kann, wobei das Gerüst eine Vielzahl von sich in einer Längsrichtung des Gerüstes erstreckende Kettfäden (30) und eine Vielzahl von sich quer zu den Kettfäden erstreckende Schussfäden (32) umfasst, wobei ein Bereich (34) des Gerüstes zwischen sich gegenüberliegenden Enden (36, 38) desselben frei von Schussfäden ist, und wobei das Gerüst so geformt ist, dass es einen Innenraum (40) definiert;
eine innere Komponente (24), welche behandelt ist, um das Innenwachstum von Gewebe nach einer Implantation zu fördern, wobei die innere Komponente innerhalb des durch das Gerüst definierten Innenraums angeordnet ist; und
ein Kniegelenk (28) mit einer knochenzugewandten Oberfläche (44);
bei der das Gerüst in dem Schussfäden-freien Bereich umgelegt ist, um ein Auge (46) zu bilden, welches das Kniegelenk so aufnimmt, dass die Kettfäden in dem Schussfäden-freien Bereich des Gerüstes um einen Teil des Umfanges des Kniegelenkes herumführen, wobei das Gerüst und die innere Komponente in dem Knochenkanal durch Kontakt zwischen der knochenzugewandten Oberfläche des Kniegelenkes und der Oberfläche des Knochens sicherbar sind.

2. Anordnung nach Anspruch 1, bei der die innere Komponente in dem Innenraum des Gerüstes derart angeordnet ist, dass eine relative Bewegung zwischen der inneren Komponente und dem Gerüst zugelassen ist.

3. Anordnung nach Anspruch 2, bei der die innere Komponente derart in dem Innenraum des Gerüstes angeordnet ist, dass eine freie Bewegung der inneren Komponente möglich ist, nur eingeschränkt durch eine Wand oder durch Wände des Gerüstes.

4. Anordnung nach einem der vorhergehenden Ansprüche, bei der das Gerüst, welches die tragende Komponente der Anordnung ist, im Wesentlichen rohrförmig ist.

5. Anordnung nach einem der vorhergehenden Ansprüche, umfassend einen Kordelzug (52), um das Gerüst und die innere Komponente durch den Knochenkanal zu ziehen.

6. Anordnung nach einem der vorhergehenden Ansprüche, bei der das Kniegelenk in dem Auge eingesetzt ist und das Gerüst relativ zu dem Kniegelenk gesichert ist, so dass eine Entfernung des Kniegelenks von dem Auge verhindert ist.

7. Anordnung nach Anspruch 6, bei der das Gerüst mittels einer sich um das Gerüst quer zu einer Hauptachse des Gerüstes erstreckende Garnumwicklung (50) gesichert ist, wobei die Garnumwicklung benachbart zu dem Kniegelenk angeordnet ist.

8. Anordnung nach Anspruch 6, bei der das Kniegelenk mittels einer um das Gerüst angeordneten Manschette (50a) gesichert ist, wobei die Manschette aus einer Gewebebindung besteht, welche eine größere Dichte als zumindest ein Hauptanteil des Gerüstes aufweist.

9. Anordnung nach einem der vorhergehenden Ansprüche, bei der die innere Komponente ein nichtgewebtes Material aufweist und einem Behandlungsvorgang ausgesetzt ist, welcher sie hydrophil macht, um so Gewebe-Innenwachstum zu fördern.

10. Anordnung noch einem der vorhergehenden Ansprüche, bei der das Kniegelenk erste und zweite Enden (72, 74) und einen zwischen den Enden angeordneten Mittelbereich (76) umfasst, wobei der Mittelbereich eine kleinere Breite (w₂) als diejenige der ersten und zweiten Enden (w₁) aufweist, so dass er den Schussfäden-freien Bereich des Gerüstes aufnehmen kann.

11. Anordnung nach Anspruch 10, bei der:
die ersten und zweiten Enden des Kniegelenks innere Oberflächen umfassen, welche gemeinsam die knochenzugewandte Oberfläche des Kniegelenkes definieren:
die ersten und zweiten Enden des Kniegelenkes den jeweiligen inneren Oberflächen gegenüberliegende äußere Oberflächen (79, 81) umfassen; und
die äußeren Oberflächen bogenförmig sind.

12. Anordnung nach Anspruch 11, bei der die äußeren Oberflächen der ersten und zweiten Enden sich in mehr als einer Ebene krümmen.

13. Anordnung nach einem der Ansprüche 10 bis 12, bei der der Mittelbereich des Kniegelenkes eine Höhe (h₂) aufweist, welche kleiner als diejenige der ersten und zweiten Enden (h₁) ist, um eine Eintiefung (77) zu definieren, in welcher der Schussfäden-freie Bereich des Gerüstes sitzen kann, wenn das Kniegelenk in dem Auge angeordnet ist und die der Entfernung des Kniegelenkes von dem Auge widersteht und das Profil der Anordnung reduziert.

14. Anordnung nach einem der Ansprüche 10 bis 13, bei der das Kniegelenk ein Passelement (88) umfasst, welches von der knochenzugewandten Oberfläche vorsteht und welches sich im Gebrauch in einer Öffnung (60) des Knochenkanals einpasst.

15. Anordnung nach Anspruch 14, bei der das Passelement eine Angrenzungsfläche (90) definiert, welche zur Angrenzung an einer Innenwand des Knochenkanal geformt ist, und bei der das Passelement sich in einer Richtung weg von der knochenzugewandten Oberfläche des Kniegelenkes hin verjüngt.

## Revendications

1. Ensemble ligament synthétique (22) comprenant :
une structure de ligament tissée allongée (26) qui peut être située dans un tunnel (18, 20) dans un os (12, 14), la structure comprenant une pluralité de chaînes (30) s'étendant dans une direction de longueur de la structure et une pluralité de trames (32) s'étendant transversalement aux chaînes, une région (34) de la structure entre des extrémités opposées (36, 38) de celle-ci étant exempte de trames, et la structure étant conformée de sorte qu'elle définisse un espace interne (40) ;
un composant interne (24) qui est traité pour favoriser la croissance interne de tissu après l'implantation, le composant interne situé dans l'espace interne défini par la structure ; et
un élément formant cabillot (28) ayant une surface en regard de l'os (44) ;
dans lequel la structure est pliée dans la région sans trame pour former un oeil (46) qui reçoit l'élément formant cabillot de sorte que les chaînes dans la région sans trame de la structure passent autour d'une partie d'un périmètre de l'élément formant cabillot, la structure et le composant interne pouvant être fixés dans le tunnel osseux par contact entre la surface en regard de l'os de l'élément formant cabillot et une surface de l'os.

2. Ensemble tel que revendiqué dans la revendication 1, dans lequel le composant interne est situé dans l'espace interne de la structure de telle sorte qu'un mouvement relatif entre le composant interne et la structure est autorisé.

3. Ensemble tel que revendiqué dans la revendication 2, dans lequel le composant interne est situé dans l'espace interne dans la structure de telle sorte qu'un libre mouvement du composant interne est possible, limité uniquement par une paroi ou des parois de la structure.

4. Ensemble tel que revendiqué dans l'une des revendications précédentes, dans lequel la structure, qui est le composant principal porteur de charge de l'ensemble, est généralement tubulaire.

5. Ensemble tel que revendiqué dans l'une des revendications précédentes, comprenant un cordon de serrage (52) pour tirer la structure et le composant interne à travers le tunnel osseux.

6. Ensemble tel que revendiqué dans l'une des revendications précédentes, dans lequel l'élément formant cabillot est inséré dans l'oeil et la structure est fixée par rapport à l'élément formant cabillot de manière à empêcher le retrait de l'élément formant cabillot de l'oeil.

7. Ensemble tel que revendiqué dans la revendication 6, dans lequel la structure est fixée au moyen d'une sangle (50) s'étendant autour de la structure transversalement à un axe principal de la structure, la sangle étant positionnée à proximité de l'élément formant cabillot.

8. Ensemble tel que revendiqué dans la revendication 6, dans lequel l'élément formant cabillot est fixé au moyen d'un manchon (50a) positionné autour de la structure, le manchon ayant un tissage présentant une densité supérieure à au moins celle d'une partie principale de la structure.

9. Ensemble tel que revendiqué dans l'une des revendications précédentes, dans lequel le composant interne est réalisé en un matériau non tissé et est soumis à un procédé de traitement qui le rend hydrophile, pour favoriser ainsi la croissance interne de tissu.

10. Ensemble tel que revendiqué dans l'une des revendications précédentes, dans lequel l'élément formant cabillot comprend des première et deuxième extrémités (72, 74) et une partie de taille (76) positionnée entre les extrémités, la partie de taille ayant une largeur (w₂) inférieure à celle des première et deuxième extrémités (w₁) de sorte qu'elle peut recevoir la région sans trame de la structure.

11. Ensemble tel que revendiqué dans la revendication 10, dans lequel :
les première et deuxième extrémités de l'élément formant cabillot comprennent des surfaces internes qui définissent, ensemble, la surface en regard de l'os de l'élément formant cabillot ;
les première et deuxième extrémités de l'élément formant cabillot comprennent des surfaces externes (79, 81), opposées aux surfaces internes respectives ; et
les surfaces externes sont arquées.

12. Ensemble tel que revendiqué dans la revendication 11, dans lequel les surfaces externes des première et deuxième extrémités se courbent dans plus d'un plan.

13. Ensemble tel que revendiqué dans l'une quelconque des revendications 10 à 12, dans lequel la partie de taille de l'élément formant cabillot a une hauteur (h₂) qui est inférieure à celle des première et deuxième extrémités (h₁) pour définir un évidement (77) où la région sans trame de la structure peut se situer lorsque l'élément formant cabillot est positionné dans l'oeil, résistant au retrait de l'élément formant cabillot de l'oeil et réduisant le profil de l'ensemble.

14. Ensemble tel que revendiqué dans l'une quelconque des revendications 10 à 13, dans lequel l'élément formant cabillot comprend un élément de positionnement (88) qui s'étend depuis la surface en regard de l'os et qui, en cours d'utilisation, se situe dans une ouverture (60) du tunnel osseux.

15. Ensemble tel que revendiqué dans la revendication 14, dans lequel l'élément de positionnement définit une surface de butée (90) qui est conformée pour venir en butée contre une paroi interne du tunnel osseux, et dans lequel l'élément de positionnement s'effile dans une direction s'éloignant de la surface en regard de l'os de l'élément formant cabillot.
